# EUROPEAN PATENT APPLICATION

(11) **EP 2 540 272 A1**
(43) Date of publication of application: **02.01.2013**
(21) Application number: 11005188.5
(22) Date of filing: 27.06.2011
(51) Int. Cl.: A61F 13/56, A61F 13/62, A61F 13/514, A61L 15/58, C09J 123/00, C09J 123/14, C09J 201/00

(54) **Multilayer material for a diaper landing zone and production method**

(71) Applicant: MAGIS S.P.A., 50050 Cerreto Guidi (FI) (IT)
(72) Inventor: Marzi, Marco, 50053 Empoli, (FI) (IT); Mannucci, Vanni, 50139 Firenze (IT)
(74) Representative: Barberi, Vittorio

(57) **Abstract**

The invention relates to a multilayer material (1) and to a method of manufacturing a multilayer material; the multilayer material (1) is usable for forming the anchoring area or "landing zone" for diapers and similar items; the material comprises: - a first layer (2) consisting of a film of polyolefin or biodegradable PLA type; - a second layer (3) of non-woven fabric, preferably of polyolefin or biodegradable PLA type; said first (2) and second (3) layers being bound together by means of a glue (4).

## Description

The present invention relates to a multilayer material and its manufacturing process.

In particular, the multilayer material is usable in the production of diapers and other items for personal hygiene, for forming the front of the diaper, in jargon called "landing zone". The landing zone is the front area of the diaper, which defines a fixing zone for a releasable coupling device of the type called "hook and loop", forming the female part of the device, the part that supports loops.

The material used for the formation of the landing areas is usually multilayered, with a top layer provided with loops in order to define the female part of the releasable closing device.

In the most usual form, the landing areas are made of a layered material obtained by a process that can be relatively complex and expensive if compared to the final cost of the product; this process in many cases can be dangerous for the health of the workers.

The aim of the present invention is to provide a new multilayered material and an item made of such material, in order to eliminate the drawbacks of the known solutions and at the same time to provide a new construction method that combine a relatively low production cost with a product which is easily and substantially completely recyclable, whose production is not harmful to operators, and which is provided with a high comfort and production simplicity. This result was achieved in accordance with the invention by adopting the idea of a material, a method and an article having the characteristics described in the independent claims. Other features are described in the dependent claims.

Among the advantages of the present invention are the following:
- the material is almost totally bio-degradable;
- the manufacturing process of the material uses materials that are not harmful to workers;
- the manufacturing process of the material is extremely simple and has relatively low costs; in addition, the construction process is relatively simple and therefore can be implemented without requiring a skilled workforce;
- the items made according to the present invention have a duration and a similar fit (certainly not worse) in respect to the traditional items.

Every technician who works in this field will better understand these advantages and features and further advantages and features of the present invention thanks to the enclosed drawings as a practical explanation of the present invention which should not be considered in a limitative sense, wherein:
- Fig.1 is a schematic perspective view of a diaper provided with the "landing zone";
- Fig.2 is a schematic sectional view of a possible embodiment of a multilayered material according to the invention.

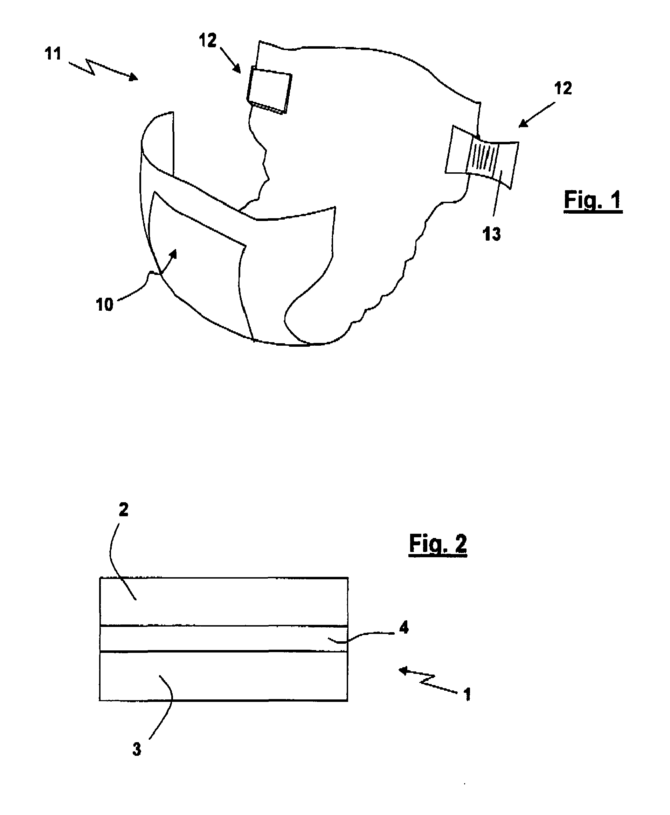

As mentioned above, the present invention relates to a multilayered material (1) usable in the production of diapers and other items for personal hygiene for forming the frontal part called "landing zone". In Fig.1, the landing zone is marked with (10) and it is placed at the front of the diaper (11). The landing zone (10) is the female part (provided with "loops") of a releasable closure device (12), provided with a respective male part (13), equipped with "hooks".

In accordance with the invention, the material (1) used for the formation of the landing zone is of multilayer type, with a top layer (2) provided with loops to define the female part of the releasable closing device.

The multilayered material according to the invention comprises;
- a first layer (2) consisting of a film, preferably of polyolefin type;
- a second layer (3) of non-woven fabric, preferably of polyolefin type;
- said first (2) and second (3) layers being bound together by means of a glue (4).

In particular, the first layer (2) can be formed by a plastic film such as polypropylene, the second layer (3) by non-woven fabric such as polypropylene, and the glue (4) interposed between the first (2) and the second layer (3) can be of polyolefin base.

The first layer may be formed by a biodegradable film (according to DIN EN 13432:2000-12 or similar), with PLA base (polylactic acid) or similar.

Similarly, the second layer can be formed by a nonwoven biodegradable fabric (according to DIN EN 13432:2000-12 or similar), with PLA base (polylactic acid) or similar.

The glue (4), which can be with polyolefin base, can be with polypropylene base.

The fixing of the first (2) and the second layer (3) can be obtained with a layer of glue (4) interposed between the two layers (2) and (3) and by means of the passage through a pair of rollers or calanders.

The glue (4) can be of reactivable thermo-adhesive type in order to simplify and optimize the bonding operations of the layers.

As mentioned above, the materials used for forming the multilayered material can be biodegradable and / or biocompatible; in this way, the disposal of nappies will be extremely consistent from the ecological point of view.

In addition, using a glue free by the presence of isocyanates during the production better working conditions are guaranteed for workers.

In accordance with the invention, a method for the formation of the layered material (1) is characterized in that a first layer (2) formed by a film of polypropylene and a second layer (3) of non-woven fabric, preferably of polypropilene, are joined by passing through a pair of rollers or calanders, between said first and second layers being interposed a layer of glue with base of polypropylene.

The materials used for the first layer (2), the second layer (3) and the glue (4) have been previously indicated. In practice, the construction details may vary in any equivalent way as regards the shape, dimensions, disposition of elements, nature of the used material, without nevertheless departing from the scope of the adopted solution idea and thereby remaining within the limits of the protection granted to the present patent.

## Claims

1. Multilayer material usable for forming the anchoring area or "landing zone" for diapers and similar items, **characterized in that** it comprises:
- a first layer (2) consisting of a film of polyolefin type;
- a second layer (3) of non-woven fabric, preferably of polyolefin type;
said first (2) and second (3) layers being bound together by means of a glue (4).

2. Multilayer material according to claim 1, **characterized in that**:
- said first layer (2) is a film of a material selected between polyethylene and polypropylene;
- said second layer (3) is a non-woven fabric of polypropylene;
- said glue is made of polyolefin.

3. Multilayer material according to claim 1, **characterized in that** said glue is made of polypropylene.

4. Multilayer material according to claim 1, **characterized in that** the first layer is a biodegradable film (according to DIN EN 13432:2000-12 or similar) with PLA base (polylactic acid) or similar.

5. Multilayer material according to claim 1, **characterized in that** the second layer is a nonwoven biodegradable fabric (according to DIN EN 13432:2000-12 or similar) with PLA base (polylactic acid) or similar.

6. Multilayer material according to claim 1, **characterized in that** said glue is a reactivable thermo-adhesive glue.

7. Method for the formation of a multilayered material usable for forming the anchorage area, or "landing zone" for diapers and the like, **characterized in that** a first layer (2) formed by a film preferably of polyolefin type and a second layer (3) of non-woven fabric, preferably of polyolefin type, are joined by passing through a pair of rollers or calanders, between said first and second layers being interposed a layer of glue.

8. Method according to claim 7, **characterized in that** said glue is a polyolefin.

9. Method according to claim 7, **characterized in that** the first layer is a biodegradable film (according to DIN EN 13432:2000-12 or similar) with PLA base (polylactic acid) or similar.

10. Method according to claim 7, **characterized in that** the second layer is a nonwoven biodegradable (according to DIN EN 13432:2000-12 or similar) with PLA base (polylactic acid) or similar.

11. Method according to claim 7, **characterized in that** said glue is a reactivable thermo-adhesive glue.
